# EUROPÄISCHE PATENTSCHRIFT

(11) **EP 0 653 414 B1**
(45) Veröffentlichungstag und Bekanntmachung des Hinweises auf die Patenterteilung: **17.09.1997**
(21) Anmeldenummer: 94117329.6
(22) Anmeldetag: 03.11.1994
(51) Int. Cl.: C07C 67/14, C07C 69/78

(54) **Verfahren zur Herstellung von Fluorbenzoesäurealkylestern durch Reaktion eines Fluorbenzoylchlorids mit einem Alkohol in Gegenwart eines Alkalimetallalkoholates**
Process for preparing alkyl esters of fluorbenzoic acid by reaction of a fluor-benzoylchlorides with an alcohol in the presence of an alkalimetal alcoholate
Procédé pour la préparation d'esters alkyliques d'acide benzoique fluoré par réaction d'un chlorure d'acide benzoique fluoré avec un alcool en présence d'un alcoolate d'un métal alcalin

(30) Priorität: 17.11.1993 DE 4339208
(43) Veröffentlichungstag der Anmeldung: 17.05.1995
(73) Patentinhaber: HOECHST AKTIENGESELLSCHAFT, 65929 Frankfurt am Main (DE)
(72) Erfinder: Billeb, Gilbert, Dr., D-65779 Kelkheim (DE); Tronich, Wolfgang, Dr., D-65817 Eppstein (DE)

(56) Entgegenhaltungen:
- DATABASE WPI Week 8850, Derwent Publications Ltd., London, GB; AN 88-357180 & JP-A-63 267 745 (NIHON NOYAKU KK) 4. November 1988
- DATABASE WPI Week 9312, Derwent Publications Ltd., London, GB; AN 93-096743 & JP-A-5 039 233 (OTSUKA PHARM CO LTD) 19. Februar 1993

## Beschreibung

Fluorbenzoesäurealkylester der allgemeinen Formel (I) sind wichtige Zwischenprodukte für die Herstellung von Pharmazeutika und Pflanzenschutzmitteln.

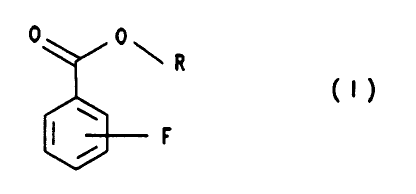

In der Literatur sind einige Verfahren zur Herstellung solcher Ester beschrieben. So erhält man 3-Fluorbenzoesäureethyl- und -methylester aus dem Silbersalz der Säure (Paterno et al., Gazz. Chim. Ital. 12, 90). Dieses Verfahren ist aber wegen Verwendung eines teuren Silbersalzes bestenfalls zur Herstellung von Mengen im Labormaßstab geeignet.

4-Fluorbenzoesäureethylester und der entsprechende Methylester entstehen bei der Umsetzung von 4-Fluorbenzoesäure mit Ethanol bzw. Methanol unter Einleiten von Chlorwasserstoffgas (Schmitt et al., J. Prakt. Chem. 1 (2), 400; Bacon et al., J. Org. Chem. 3, 281 (1938); Bowden et al., H. Chem. Soc. 1940, 1249). Diese Reaktion liefert bei langen Reaktionszeiten nur mäßige Ausbeuten und ist außerdem aufgrund der als Nebenreaktion ablaufenden Bildung von Alkylhalogeniden durch Reaktion von Chlorwasserstoffgas mit den Alkoholen von Nachteil (vgl. Beispiel 1).

2- und 4-Fluorbenzoesäuremethyl- und -ethylester erhält man weiterhin durch Erhitzen von 2- bzw. 4-Methoxycarbonylphenyldiazoniumtetrafluoroborat (Bergmann et al., Chem. Ber. 64, 1455 (1931); Schiemann et al., Org. Synth. Coll. Vol. II, 299 (1943); Jap. Patentschrift 05039233). Da die hierfür benötigten Ausgangsstoffe nur mit großem Aufwand zugänglich sind, sind diese Reaktionen technisch nicht leicht durchführbar und liefern zudem meist nur mäßige Ausbeuten.

Nur für Labormengen geeignete Varianten dieser Reaktion sind die Umsetzung von Ethylnitrit mit 4-Aminobenzoesäureethylhexafluorosilikat (Wiley et al., J. Am. Chem. Soc. 71, 1863 (1949)) und die Umsetzung von 2-Aminobenzoesäuremethylester mit Nitrosoniumtetrafluoroborat (Milner et al., Synth. Commun. 22 (1), 73 (1992)).

4-Fluorbenzoesäuremethylester kann auch durch Pd-katalysierte Alkoxycarbonylierung von 4-Iodofluorbenzol, welches technisch zu nicht akzeptablen Preisen verfügbar ist, hergestellt werden (Carpentier et al., Tetrahedron Lett. 32 (36), 4705 (1991)). Hierbei erhält man aber ca. 9 % an 4-Methoxybenzoesäuremethylester als Nebenprodukt. Varianten dieser Reaktion sind die Umsetzungen von 4-Iodofluorbenzol mit Ethanol und CO an Zeolith-Katalsytoren oder under Pd-Katalyse, die aber nur 62 bzw. 76 % Ausbeute liefern (JP 04046139; JP 03197441), sowie die Reaktion von 4-Fluorbrombenzol mit Methyliodid und CO an Kobaltkatalysatoren (Itoh et al., Mol. Catal. 48 (1), 11 (1988)).

Nur für den Labormaßstab geeignete Herstellmethoden für Fluorbenzoesäurealkylester sind die elektrochemische Reduktion von 2,6-Difluorbenzoesäureestern (Hebri et al., Synth. Commun. 21 (22), 2377 (1991)) und die anodische Oxidation von Essigsäure-2-(4-fluorbenzoat) (4-F-C₆H₄COOCH₂COOH) (Thomas et al., Chem. Ber. 118 (7), 2777 (1985)).

In der Literatur sind ferner Verfahren zur Herstellung von 4-Fluorbenzoesäurealkylestern durch Umsetzung der entsprechenden Fluorbenzoesäurechloride mit Alkoholen beschrieben (FR 2541282; Smith et al., J. Am. Chem. Soc. 79, 875 (1957); Spratt et al., Anal. Chem. 56 (12), 2038 (1984)). Bei dieser Reaktion findet jedoch in der Regel als Nebenreaktion Alkylchlorid-Bildung statt (vgl. Beispiel 1), was die Ausbeute erniedrigt und zu ökologisch bedenklicher Prozeßabluft führt. Außerdem verläuft diese Reaktion oft langsam und erfordert höhere Temperaturen, welche die Alkylchloridbildung noch begünstigen. Die Verwendung von Aminen als Lösungsmittel als Katalysator, wie in diesen Literaturstellen beschrieben, beschleunigt zwar die Reaktion, ist aber aufgrund der Bildung von Aminhydrochloriden aus dem Amin und der entstehenden Salzsäure technisch ungünstig. Die Hydrochloride müßten aufwendig zu freiem Amin regeneriert werden. Außerdem ist die Produktqualität dann in der Regel so schlecht, daß eine aufwendige destillative Aufarbeitung notwendig wird (vgl. Beispiel 2).

Daher bestand ein sehr hoher Bedarf nach einem neuen Verfahren zur Herstellung von Fluorbenzoesäurealkylestern, das die beschriebenen Nachteile nicht aufweist, von leicht zugänglichen Stoffen ausgeht, die gewünschten Verbindungen in hoher Ausbeute zugänglich macht und sich zudem technisch ohne großen Aufwand realisieren läßt.

Diese Aufgabe wird gelöst durch ein Verfahren zur Herstellung von Fluorbenzoesäurealkylestern der allgemeinen Formel (I) 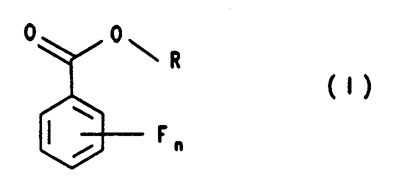 worin R für einen Alkylrest und Fₙ für 1 bis 4 Fluoratome, die unabhängig voneinander an den aromatischen Ring gebunden sind, steht, indem man ein Fluorbenzoylchlorid der allgemeinen Formel II 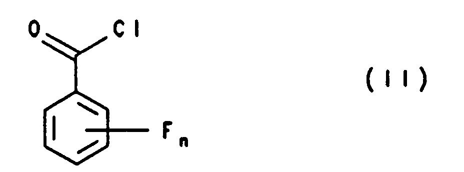 in der Fₙ die oben definierte Bedeutung hat mit einer mindestens stöchiometrischen Menge eines Alkalialkoholates ROM,
worin R die oben definierte Bedeutung hat und M für ein Alkalimetall der Gruppe Li, Na, K steht, in dem entsprechenden Alkohol ROH bei Temperaturen von -10°C bis 150°C umsetzt, das Lösungsmittel gegebenenfalls abdestilliert und durch Behandeln mit Wasser oder Filtration das gebildete Alkalichlorid abtrennt.

Die Reaktion wird zweckmäßig so geführt, daß man pro Mol umzusetzendes Säurechlorid 1,0 bis 5,0 Mol, insbesondere 1,0 bis 3,0, bevorzugt 1,0 bis 1,5 Mol Alkoholat einsetzt. Dies wird vorteilhaft in einer solchen Menge des entsprechenden Alkoholes gelöst vorgelegt, daß der Alkoholatgehalt der Lösung zwischen 1 und 60 %, insbesondere 10 bis 50 %, bevorzugt zwischen 20 und 40 % liegt. Das Säurechlorid wird, gegebenenfalls unter Kühlung, bei einer Temperatur von -10°C bis Siedetemperatur des Alkohols, insbesondere 10°C bis Siedetemperatur des Alkohols, zugetropft. Anschließend wird bei -10°C bis Rückflußtemperatur des Alkohols, insbesondere 10°C bis Rückflußtemperatur des Alkohols, bevorzugt bei 10°C bis 40°C nachgerührt. Es hat sich in vielen Fällen bewährt, das Reaktionsgemisch mit Wasser zu verdünnen und das Produkt durch Phasentrennung oder Filtration zu isolieren. Hierbei hat es sich als günstig erwiesen, vor der Wasserzugabe überschüssigen Alkohol abzudestillieren.
Eine weitere Möglichkeit besteht darin, das Produkt durch Abfiltrieren des Alkalisalzes und nachfolgende destillative Abtrennung des Alkohols zu isolieren.

Dieses Verfahren zeichnet sich somit gegenüber den literaturbekannten Verfahren durch hohe Ausbeuten aus, wobei ohne Reinigung der Rohprodukte hohe Reinheiten erzielt werden. Besonders überraschend bei diesem Verfahren ist, daß der befürchtete nucleophile Fluor-Methoxy-Austausch, wie er z. B. bei Williams et al., J. Org. Chem. 42, 3414 (1977), James et al., J. Fluorine Chem. 27 (N1), 91 (1985) und vielen anderen Literaturstellen beschrieben wird, nicht auftritt. Bei diesem Verfahren werden auch keine Alkylhalogenide gebildet, so daß keine besonderen Anforderungen an die Abluftreinigung gestellt werden müssen. Die Edukte sind billig und in großtechnischen Mengen verfügbar. 3-Fluorbenzoylchlorid wird z. B. durch Seitenkettenchlorierung von 3-Fluortoluol und nachfolgender Verseifung oder durch Umsetzung von 3-Fluorbenzoesäure mit Thionylchlorid hergestellt. Die Raum-Zeit-Ausbeuten sind auf allen Stufen hoch.

Die nachfolgenden Beispiele dienen zur Erläuterung des Verfahrens, ohne es darauf zu beschränken.

### Beispiele

Die für die Vergleichsbeispiele verwendeten Fluorbenzoesäurechloride wurden durch Seitenkettenchlorierung der entsprechenden Fluortoluole und nachfolgende Verseifung hergestellt.

### Beispiel 1 (Vergleichsbeispiel):

### Umsetzung von Alkoholen mit 3-Fluorbenzoylchlorid

a) 600,0 g (3,78 Mol) 3-Fluorbenzoylchlorid werden bei Raumtemperatur vorgelegt. Innerhalb von 3 h werden 130,0 g (4,06 Mol) Methanol zugetropft, wobei die Temperatur durch Kühlen bei 30°C gehalten wird. Nach ca. 15 Min. setzte die Entwicklung von Chlorwasserstoff ein. Während der Dosierung werden im Abgas gaschromatographisch >10 Vol.-% Methylchlorid nachgewiesen. Nach Ende der Dosierung wird 15 Min. bei 30°C und anschließend zum Erreichen eines vollständigen Umsatzes 30 Min. bei Rückflußtemperatur nachgerührt. Dann wird überschüssiges Methanol und gelöste Salzsäure abdestilliert.
   Ausbeute: 537,1 g (3,48 Mol) 3-Fluorbenzoesäuremethylester ≙ 92,2 % d. Th..
b) 638,0 g (4,02 Mol) 3-Fluorbenzoylchlorid werden bei Raumtemperatur vorgelegt. Innerhalb von 2 h werden 195,0 g (4,22 Mol) Ethanol zugetropft, wobei die Temperatur auf 52°C ansteigt. Nach ca. 15 Min. setzt die Chlorwasserstoff-Entwicklung ein. Während der Dosierung lassen sich im Abgas >0,5 Vol.-% Ethylchlorid nachweisen. Anschließend wurde 12 h bei 55°C nachgerührt, ohne daß vollständiger Umsatz an Säurechlorid erreicht wird. Nach weiteren 2 h bei 95°C ist der Umsatz quantitativ. Durch Destillation im Vakuum bei 40°C wird überschüssiger Alkohol und Chlorwasserstoff entfernt.
   Ausbeute: 642,1 g (3,82 Mol) 3-Fluorbenzoesäureethylester ≙ 95 % d. Th..
c) In weiteren Versuchen werden Methanol bzw. Ethanol vorgelegt und das Säurechlorid zudosiert, sowie Temperaturen und Zeiten variiert, wobei stets die Bildung von Methyl- bzw. Ethylchlorid beobachtet wurde. Die Ausbeute verbessert sich nicht.

### Beispiel 2 (Vergleichsbeispiel):

### Umsetzung in Gegenwart von Tributylamin

112,0 g (3,5 Mol) Methanol und 583,0 g (3,15 Mol) Tri-n-butylamin werden bei Raumtemperatur vorgelegt. Innerhalb von 2 h werden 500,0 g (3,15 Mol) 3-Fluorbenzoylchlorid bei Raumtemperatur zugetropft. Es wird kein Abgas beobachtet. Nach beendeter Dosierung wird 20 min. bei 80°C nachgerührt. Nach dieser Zeit ist der Umsatz quantitativ. Es wird mit 1500 ml Wasser und 100 ml konz. Hcl verdünnt. Die organische Phase wird abgetrennt und mit 100 ml Wasser neutral gewaschen.
Ausbeute: 468,2 g roher 3-Fluorbenzoesäuremethylester (= 88,2 d. Th. 100 % ger.) mit einem Reingehalt von 91,5 % und einem Gehalt an Tributylamin von 6,4 %. Durch alkalisch Stellen der wäßrigen Phase und nachfolgender Phasentrennung werden 600 g wasserhaltiges Tributylamin zurückgewonnen, welches für weitere Versuche jedoch zunächst noch getrocknet werden muß.

### Beispiel 3:

### Umsetzung von Natriummethylat mit 3-Fluorbenzoylchlorid

a) 1427 g (7,66 Mol) einer 29 %igen Lösung von Natriummethanolat in Methanol werden bei Raumtemperatur vorgelegt und innerhalb von 3 h 1200 g (7,56 Mol) wird 3-Fluorbenzoylchlorid zugetropft, wobei die Temperatur durch Kühlen bei Raumtemperatur gehalten wird. Es bildet sich eine farblose Kristallsuspension. Es wird kein Abgas beobachtet. Der Methylchloridgehalt im Gasraum des Kolbens liegt weit unterhalb von 0,1 Vol.-%. Es wird 30 min. bei Raumtemperatur nachgerührt. Nach dieser Zeit war der Umsatz quantitativ. Anschließend wird überschüssiges Methanol bei Normaldruck abdestilliert und der verbleibende Reaktionssumpf mit 1500 g Wasser versetzt. Die organische Produktphase wird abgetrennt.
   Ausbeute: 1131 g (7,33 Mol) 3-Fluorbenzoesäuremethylester = 97 % d. Th..
   Reingehalt: 99,9 % (GC, org. Bestandteile), Wassergehalt 0,5 %.
b) Ein analoges Experiment bei Rückflußtemperatur ergab gleiche Ergebnisse.

### Beispiel 4 bis 9:

### Weitere Umsetzungen

Analog dem Beispiel 3a) wurden folgende Umsetzungen durchgeführt:

### Beispiel 4:

Die Umsetzung von 3-Fluorbenzoylchlorid mit Natriumethanolat in Ethanol analog Beispiel 3a) ergibt eine Ausbeute von 97 % d. Th. bei einem Reingehalt von 99,8 % (GC), Wassergehalt 0,4 %.

### Beispiel 5:

Die Umsetzung von 3-Fluorbenzoylchlorid mit natrium-n-propanolat in n-Propanol analog Beispiel 3a) ergibt eine Ausbeute von 97 % d. Th. bei einem Reingehalt von 99, 8 % (GC), Wassergehalt 0,5 %.

### Beispiel 6:

Die Umsetzung von 3-Fluorbenzoylchlorid mit Natrium-i-propanolat in i-Propanol analog Beispiel 3a) ergibt eine Ausbeute von 96 % d. Th. bei einem Reingehalt von 99,7 % (GC), Wassergehalt 0,5 %.

### Beispiel 7:

Die Umsetzung von 4-Fluorbenzoylchlorid mit Natriummethanolat in Methanol analog Beispiel 3a) ergibt eine Ausbeute von 97 % d. Th. bei einem Reingehalt von 99,8 % (GC), Wassergehalt 0,4 %.

### Beispiel 8:

Die Umsetzung von 3-Fluorbenzoylchlorid mit Natriummethanolat in Methanol wird analog Beispiel 3a) durchgeführt, wobei aber vor Abtrennen des überschüssigen Alkohols das Kochsalz abfiltriert wird. Nach der Abtrennung des Alkohols fällt das Produkt in gleicher Ausbeute und Qualität an.

### Beispiel 9:

Die Umsetzung von 2,6-Difluorbenzoylchlorid mit Natriummethanolat in Methanol analog Beispiel 3a) ergibt eine Ausbeute von 94 % d. Th. bei einem Reingehalt von 99,7 % (GC), Wassergehalt 0,5 %.

### Beispiel 10:

Die Umsetzung von 2,4-Difluorbenzoylchlorid mit Natriummethanolat in Methanol analog Beispiel 3a) ergibt eine Ausbeute von 96 % d. Th. bei einem Reingehalt von 99,5 % (GC), Wassergehalt 0,6 %.

## Patentansprüche

1. Verfahren zur Herstellung von Fluorbenzoesäurealkylestern der allgemeinen Formel (I) 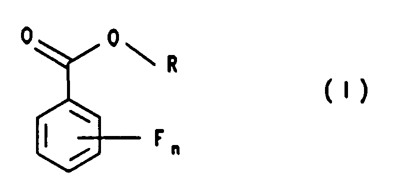 worin R für einen Alkylrest und Fₙ für 1 bis 4 Fluoratome, die unabhängig voneinander an den aromatischen Ring gebunden sind, steht, dadurch gekennzeichnet, daß man ein Fluorbenzoylchlorid der allgemeinen Formel (II) 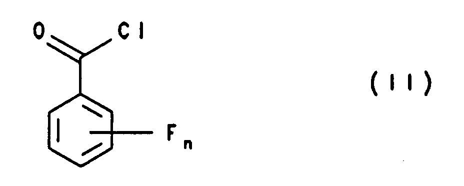 in der Fₙ die oben definierte Bedeutung hat, mit einer mindestens stöchiometrischen Menge eines Alkalimetallalkoholates ROM, worin R die oben definierte Bedeutung hat und M für ein Alkalimetall der Gruppe Li, Na, K steht, in dem entsprechenden Alkohol ROH bei Temperaturen von -10°C bis 150°C umsetzt, das Lösungsmittel gegebenenfalls abdestilliert und durch Behandeln mit Wasser oder Filtration das gebildete Alkalichlorid abtrennt.

2. Verfahren nach Anspruch 1, dadurch gekennzeichnet, daß R für Methyl, Ethyl, n-Propyl oder i-Propyl steht.

3. Verfahren nach Anspruch 1 oder 2, dadurch gekennzeichnet, daß Fₙ für 1 bis 2 Fluoratome steht.

4. Verfahren nach mindestens einem der Ansprüche 1 bis 3, dadurch gekennzeichnet, daß man das Alkalimetallalkoholat ROM im Alkohol ROH vorlegt und das Säurechlorid bei Temperaturen von -10°C bis Siedetemperatur des Alkohols, insbesondere 10°C bis Siedetemperatur des Alkohols zutropft.

5. Verfahren nach mindestens einem der Ansprüche 1 bis 4, dadurch gekennzeichnet, daß die Konzentration des Alkalimetallakoholats im Alkohol 1 bis 60 Gew.-%, insbesondere 10 bis 50 Gew.-%, bevorzugt 20 bis 40 Gew.-%, beträgt.

6. Verfahren nach mindestens einem der Ansprüche 1 bis 5, dadurch gekennzeichnet, daß man pro Mol Säurechlorid 1,0 bis 5,0 Mol, insbesondere 1,0 bis 3,0 Mol, bevorzugt 1,0 bis 1,5 Mol Alkoholat einsetzt.

7. Verfahren nach mindestens einem der Ansprüche 1 bis 6, dadurch gekennzeichnet, daß man bei -10°C bis Siedetemperatur des Alkohols, insbesondere 10°C bis Siedetemperatur des Alkohols, bevorzugt 10°C bis 40°C, nachrührt.

8. Verfahren nach mindestens einem der Ansprüche 1 bis 7, dadurch gekennzeichnet, daß man zur Aufarbeitung mit Wasser verdünnt und das Produkt durch Phasentrennung isoliert.

9. Verfahren nach mindestens einem der Ansprüche 1 bis 8, dadurch gekennzeichnet, daß man vor der Wasserzugabe überschüssigen Alkohol abdestilliert.

10. Verfahren nach mindestens einem der Ansprüche 1 bis 6, dadurch gekennzeichnet, daß man das Produkt durch Abfiltrieren des Alkalisalzes und nachfolgende destillative Abtrennung des Alkohols isoliert.

## Claims

1. A process for the preparation of alkyl fluorobenzoates of the formula (I) 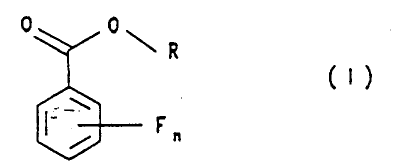 in which R is an alkyl radical and Fₙ is 1 to 4 fluorine atoms which, independently of one another, are bonded to the aromatic ring, which comprises reacting a fluorobenzoyl chloride of the formula (II) 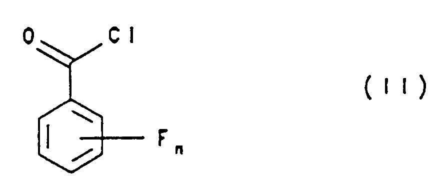 in which Fₙ is as defined above, with an at least stoichiometric amount of an alkali metal alcoholate ROM, in which R is as defined above and M is an alkali metal from the group consisting of Li, Na and K, in the corresponding alcohol ROH at temperatures from -10°C to 150°C, if appropriate distilling off the solvent, and separating off the alkali metal chloride formed by treating the mixture with water or by filtration.

2. The process as claimed in claim 1, wherein R is methyl, ethyl, n-propyl or i-propyl.

3. The process as claimed in claim 1 or 2, wherein Fₙ is 1 or 2 fluorine atoms.

4. The process as claimed in at least one of claims 1 to 3, wherein the alkali metal alcoholate ROM is introduced into the alcohol ROH and the acid chloride is added dropwise at temperatures from -10°C to the boiling point of the alcohol, in particular 10°C to the boiling point of the alcohol.

5. The process as claimed in at least one of claims 1 to 4, wherein the concentration of the alkali metal alcoholate in the alcohol is 1 to 60% by weight, in particular 10 to 50% by weight, preferably 20 to 40% by weight.

6. The process as claimed in at least one of claims 1 to 5, wherein 1.0 to 5.0 mol, in particular 1.0 to 3.0 mol, preferably 1.0 to 1.5 mol, of alcoholate are employed per mol of acid chloride.

7. The process as claimed in at least one of claims 1 to 6, wherein stirring of the mixture is continued at -10°C to boiling point of the alcohol, in particular 10°C to boiling point of the alcohol, preferably 10°C to 40°C.

8. The process as claimed in at least one of claims 1 to 7, wherein, for working-up, the mixture is diluted with water and the product is isolated by phase separation.

9. The process as claimed in at least one of claims 1 to 8, wherein excess alcohol is distilled off before water is added.

10. The process as claimed in at least one of claims 1 to 6, wherein the product is isolated by filtering off the alkali metal salt and subsequent removal of the alcohol by distillation.

## Revendications

1. Procédé pour la préparation des esters de l'acide fluorobenzoïque de formule générale (I) 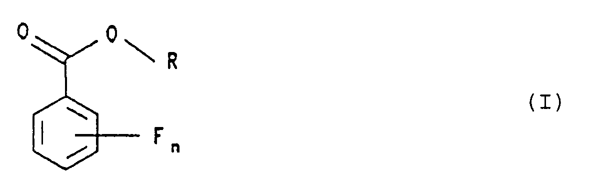 dans laquelle R représente un reste alkyle et Fₙ correspond à 1 à 4 atomes de fluor, qui sont liés indépendamment l'un de l'autre au cycle aromatique, en faisant réagir un chlorure de fluorobenzoyle de formule générale (II) 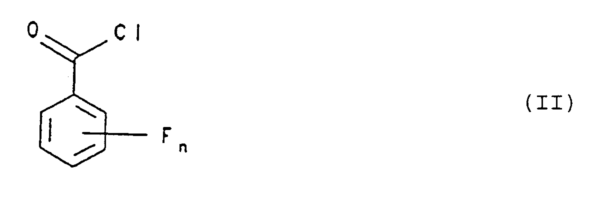 dans laquelle Fₙ a la définition donnée ci-dessus avec une quantité au moins stoechiométrique d'un alcoolate alcalin ROM, où R a la signification donnée ci-dessus et M est un métal alcalin pris dans le groupe comportant Li, Na, K, pour obtenir l'alcool ROH correspondant, à des températures de -10 °C à 150 °C, éventuellement en séparant par distillation le solvant et par traitement avec de l'eau ou par filtration le chlorure d'alcali formé.

2. Procédé selon la revendication 1, caractérisé en ce que R représente méthyle, éthyle, n-propyle ou i-propyle.

3. Procédé selon la revendication 1 ou 2, caractérisé en ce que Fₙ correspond à 1 à 2 atomes de fluor.

4. Procédé selon au moins l'une des revendications 1 à 3, caractérisé en ce que l'on prend l'alcoolate de métal alcalin ROM dans l'alcool ROH et que l'on ajoute goutte à goutte le chlorure d'acide à des températures de -10 °C à la température d'ébullition de l'alcool, notamment de 10 °C à la température d'ébullition de l'alcool.

5. Procédé selon au moins l'une des revendications 1 à 4, caractérisé en ce que la concentration de l'alcoolate de métal alcalin dans l'alcool est de 1 à 60 % en poids, plus particulièrement de 10 à 50 % en poids, de manière préférée de 20 à 40 % en poids.

6. Procédé selon au moins l'une des revendications 1 à 5, caractérisé en ce que l'on utilise par mole de chlorure d'acide de 1,0 à 5,0 moles, notamment de 1,0 à 3,0 moles, de préférence de 1,0 à 1,5 mole d'alcoolate.

7. Procédé selon au moins l'une des revendications 1 à 6, caractérisé en ce que l'on agite à une température de -10 °C à la température d'ébullition de l'alcool, notamment de 10 °C à la température d'ébullition de l'alcool, de préférence de 8 °C à 40 °C.

8. Procédé selon au moins l'une des revendications 1 à 7, caractérisé en ce que, en vue de traitement, on dilue avec de l'eau et on isole le produit par séparation de phases.

9. Procédé selon au moins l'une des revendications 1 à 8, caractérisé en ce que l'on sépare l'excès d'alcool avant l'addition de l'eau.

10. Procédé selon au moins l'une des revendications 1 à 6, caractérisé en ce que l'on isole le produit par séparation du sel de métal alcalin par filtration et ensuite par séparation de l'alcool par distillation.
